# EUROPEAN PATENT APPLICATION

(11) **EP 2 762 870 A1**
(43) Date of publication of application: **06.08.2014**
(21) Application number: 13405016.0
(22) Date of filing: 31.01.2013
(51) Int. Cl.: G01N 27/27, G01N 27/416, G01N 33/497, H04M 1/02

(54) **Sensor arrangement and portable electronic device with such a sensor arrangement**

(71) Applicant: Sensirion AG, 8712 Stäfa (CH)
(72) Inventor: Mayer, Felix, 8712 Stäfa (CH); Lechner, Moritz, 8713 Uerikon (CH); Graf, Markus, 8005 Zürich (CH)
(74) Representative: Fischer, Britta Ruth

(57) **Abstract**

The invention relates to a sensor arrangement (1) with a chemical sensor (2) for measuring a chemical property of a fluid the chemical sensor (2) is subjected to and compensation means (3) for compensating an error in the measurement signal of the chemical sensor (2) that is due to humidity, wherein the compensation means (3) comprise a humidity sensor (4), with the chemical sensor (2) and the humidity sensor (4) being arranged on the same circuit board (5), wherein the chemical sensor (2) and the humidity sensor (4) are each given by a sensor chip that comprises a sensing layer (6.2; 6.4) and a substrate layer (7.2; 7.4), with each substrate layer (7.2; 7.4) being provided with at least one via (8.2; 8.4) for electrically connecting the respective sensing layer (6.2; 6.4) with the circuit board (5), and wherein the compensation means (3) further comprise a processing unit (9) for processing the measurement signal of the chemical sensor (2) and the measurement signal of the humidity sensor (4) for error compensation, the error in the measurement signal of the chemical sensor (2) to be compensated being due to humidity. The invention further relates to a portable electronic device (10) with such a sensor arrangement (1).

## Description

### Technical Field

The invention relates to a sensor arrangement and a portable electronic device with such a sensor arrangement according to the preambles of the independent claims.

### Background Art

As described in European patent application no. 12 000 446.0 portable electronic devices such as smart phones and tablet computers may comprise a chemical sensor for measuring a property of a fluid - and thereby an analyte that forms part of the fluid - that the chemical sensor is exposed to through an opening in the housing of the portable electronic device. Such a property may, for example, be the amount of carbon dioxide or ozone in the ambient air.

However, the chemical sensors employed might respond not only to the chemical property to be determined but also to the humidity of their environment. I.e. the amount of humidity the chemical sensor is exposed to in connection with the exposure to the fluid that shall be analyzed might distort, in particular bias, the measurement signal (the output signal) of the chemical sensor leading to inaccurate measurements.

### Disclosure of the Invention

It is an object of the invention to provide a sensor arrangement with a chemical sensor by which distortion of the measurement signal of the chemical sensor due to humidity can be avoided or at least reduced and that can be employed in a portable electronic device, in particular a smart phone. It is a further object of the invention to provide a portable electronic device with such a sensor arrangement.

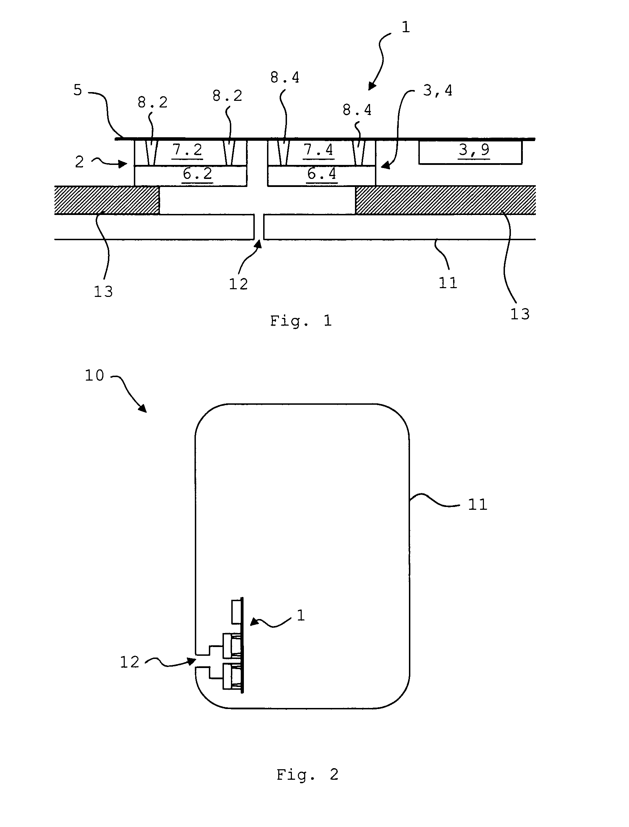

In order to implement these and still further objects of the invention, which will become more readily apparent as the description proceeds, a sensor arrangement is provided that comprises a chemical sensor for measuring a chemical property of a fluid that the chemical sensor is exposed to. The sensor arrangement furthermore comprises compensation means for compensating a possible error in the measurement signal of the chemical sensor (in the following: chemical measurement signal) that is due to humidity the chemical sensor is exposed to. The compensation means comprise a humidity sensor that is arranged on the same circuit board as the chemical sensor, preferably in close proximity or next to the chemical sensor. The chemical sensor and the humidity sensor are each given by a sensor chip comprising a sensing layer for performing the respective measuring and a substrate layer, the sensing layer being placed on top of the substrate layer. Each substrate layer is provided with at least one via, preferably at least one so-called through-silicon-via ((TSV; http://en.wikipedia.org/wiki/Through-silicon_via), for electrically connecting the respective sensing layer with the circuit board. The material of the substrate layer may for example be silicon. The expression "on top" corresponds to Figure 1 upside down.

The compensation means further comprise a processing unit for processing the chemical measurement signal and the measurement signal of the humidity sensor (in the following: humidity measurement signal). The processing unit is preferably given by a microprocessor and is preferentially mounted on the same circuit board as the chemical sensor and the humidity sensor. The processing unit processes both the measurement signal of the chemical sensor and the measurement signal of the humidity sensor. When processing the chemical measurement signal the processing unit preferably takes into account the humidity measurement signal in order to compensate a possible error in the chemical measurement signal that is due to the humidity the chemical sensor (and also the humidity sensor) is exposed to. Preferentially the actual error compensation is performed by the processing circuit. The actual (in particular mathematical/computational) error compensation may, however, also be performed by an external device to which the processing circuit transmits both measurement signals.

The processing unit is for example designed such that it subtracts the humidity measurement signal (or a signal derived therefrom) from the chemical measurement signal (or a signal derived therefrom). The measurement signals correspond to the output signals of the respective sensors.

Furthermore, the invention relates to a portable electronic device with a housing and a sensor arrangement according to the invention, wherein the sensor arrangement is arranged inside the housing beneath an opening provided in the housing, such that the sensor arrangement is subjected to fluid passing through the opening, with both the chemical sensor and the humidity sensor being subjected to the fluid. In particular, the sensor arrangement of the invention is arranged such inside the housing that the humidity within the fluid passing through the opening reaches both the chemical sensor and the humidity sensor preferentially at the same concentration. The portable electronic device may for example be one of the following: a mobile phone, a smart phone, a tablet computer, a notebook or similar.

The fluid to be analysed by the sensor arrangement according to the invention may be a fluid in the environment of the portable electronic device. The fluid may comprise a liquid or a gas and may contain one or more ingredients/analytes. The chemical sensor of the sensor arrangement is designed to measure/determine one or more properties of one or more of the ingredients/analytes of the fluid.

For cases where the fluid to be analyzed is for example a person's breath as in field sobriety tests an acoustic sensor in form of a microphone can be arranged on the circuit board next to or in close proximity to the chemical sensor and/or the humidity sensor beneath the opening in the housing. The microphone is preferably also realized as semiconductor sensor chip with a sensing layer that converts an acoustic signal to an electric signal arranged on top of a substrate layer, wherein at least one via is provided in the substrate layer for electrically connecting the sensing layer to the circuit board.

When measuring a property of a fluid by means of a chemical sensor arranged beneath an opening in a housing of a portable electronic device, the housing and the opening together with the space between opening and chemical sensor, respectively, act as low pass filter, limiting the diffusion of the fluid to be analysed to the chemical sensor and thus the response time of the chemical sensor. Reducing the dead space between the opening and the chemical sensor is such vital to achieve fast response time. This can be achieved with the sensor arrangement of the present invention.

Designing each of the sensors as sensor chip with at least one via for electrical connection, leads to both the chemical sensor and the humidity sensor being small and compact in size and hence requiring only a limited amount of space in a portable electronic device, thereby minimizing the dead space between the opening in the housing of a portable electronic device and the chemical sensor and also minimizing the space between both sensors. Preferably the lateral distance between the chemical sensor and the humidity sensor is not larger than 2 Millimetres. Such space minimizations would not be achievable if bond wires - typically enclosed by rather spacious glob-tops - were used instead of vias for electrical connection or if all sensor functions would be realized by one chip.

Moreover, as the chemical sensor, the humidity sensor and, if applicable, the acoustic sensor, are realized as separate sensor chips, the most-efficient manufacturing process and calibration process, respectively, can be employed for each sensor depending on the particular sensor type. I.e. no compromise between various manufacturing processes needs to be made, which might lead to less efficient sensors.

### Brief Description of the Drawings

Further advantageous features and applications of the invention can be found in the dependent claims, as well as in the following description of the drawings illustrating the invention. In the drawings like reference signs designate the same or similar parts/components throughout the several figures of which:
Fig. 1 shows a schematic drawing of a sensor arrangement according to the invention, and
Fig. 2 shows a schematic drawing of a portable electronic device with a sensor arrangement according to the invention.

### Mode(s) for Carrying out the Invention

Figure 1 depicts a sensor arrangement 1 according to the invention. The sensor arrangement 1 comprises a chemical sensor 2 and compensation means 3 for compensating a possible error in the chemical measurement signal due to humidity the sensor arrangement 1 is exposed to. The compensation means 3 comprise a humidity sensor 4 that is positioned next to the chemical sensor 2 on a circuit board 5. Preferably, the distance the chemical sensor 2 and the humidity sensor 4 are placed apart lies in the range from 0.01 to 10 Millimetres, in particular in the range from 0.1 to 2 Millimetres.

Both the chemical sensor 2 and the humidity sensor 4 are realized as a sensor chip with a sensing layer 6.2, 6.4 and a substrate layer 7.2, 7.4, the respective sensing layer 6.2, 6.4 being arranged on top of the respective substrate layer 7.2, 7.4. Each sensing layer 6.2, 6.4 preferably comprises a specific sensing element (not shown) for performing the measuring function. For protection of the respective sensing element each sensing layer 6.2, 6.4 may comprise a handling layer (not shown) that apart from protecting the sensing element may be used for handling (e.g. gripping or similar) of the entire sensor 2, 4 for example during manufacturing.

For electrical connection to the circuit board 5 (or for example a digital bus) the chemical sensor 2 is provided with exemplarily two vias 8.2, that extend from the side of the substrate layer 7.2 that is adjacent to the sensing layer 6.2 to the opposite side of the substrate layer 7.2 that is adjacent to the circuit board 5. The vias 8.2 electrically connect the sensing layer 6.2 of the chemical sensor 2 to the circuit board 5 such that an (electrical) measurement signal of the chemical sensor 2 is conveyed to the circuit board 5.

Similarly, the humidity sensor 4 is provided with exemplarily two vias 8.4, that extend from the side of the substrate layer 7.4 that is adjacent to the sensing layer 6.4 to the opposite side of the substrate layer 7.4 that is adjacent to the circuit board 5. The vias 8.4 electrically connect the sensing layer 6.4 of the humidity sensor 4 to the circuit board 5 such that an (electrical) measurement signal of the humidity sensor 4 is conveyed to the circuit board 5.

The vias 8.2, 8.4 are preferably isolated from the surrounding substrate 7.2, 7.4, for example by a layer of SiO₂ (not shown), and are filled with conducting material (not shown), in particular coated with conducting material on their respective inner wall, to form the electrical connection between the circuit board 5 and the respective sensing layer 6.2, 6.4.

The compensation means 3 further comprise a processing unit 9 for processing both the measurement signal of the chemical sensor 2 and measurement signal of the humidity sensor 4. The processing unit 9 is preferably mounted on the circuit board 5 as depicted in Figure 1 and may be positioned in the proximity of the chemical sensor 2 and/or the humidity sensor 4. Alternatively, the processing unit 9 may be provided separate from the circuit board 5 with the circuit board 5 being electrically connected to the processing unit 9. The processing unit 9 is preferably given by a microprocessor.

The processing unit 9 is designed such that it takes the humidity measurement signal into account for possible error compensation when processing the chemical measurement signal. I.e. the processing unit 9 is designed such that it employs the humidity measurement signal to compensate a possible error in the chemical measurement signal that has arisen due to humidity both the chemical sensor 2 and the humidity sensor 4 have been exposed to, while having been exposed to the fluid to be analysed by the chemical sensor 2.

Both the chemical sensor 2 and the humidity sensor 4 are preferably CMOS-integrated sensors, preferentially each with a bus interface. Instead or additionally to being mounted on the same circuit board both sensors 2, 4 may be connected to the same digital bus (not shown), to which preferably also the processing unit is connected.

The sensor arrangement 1 can be arranged inside a housing 11 of a portable electronic device 10 such as a smart phone (see Figure 2). For this, the sensor arrangement 1 is positioned beneath an opening 12 inside the housing 11 such that a fluid entering the opening 12 will also contact both the chemical sensor 2 and the humidity sensor 4. The opening 12 may be given by a port. For protection of the sensor arrangement 1 spacers 13 can be provided between the inner wall of the housing 11 and both the chemical sensor 2 and the humidity sensor 4 (see Figure 1). Each of the chemical sensor 2 and the humidity sensor 4 may be manufactured and/or calibrated individually and separately from the other sensor 4, 2 before they are arranged as sensor arrangement 1 inside the portable electronic device 1.

It is to be understood that while certain embodiments of the present invention have been illustrated and described herein, it is not to be limited to the specific embodiments described and shown.

## Claims

1. Sensor arrangement with a chemical sensor (2) for measuring a chemical property of a fluid the chemical sensor (2) is subjected to and compensation means (3) for compensating an error in the measurement signal of the chemical sensor (2) that is due to humidity, **characterized in that**
- the compensation means (3) comprise a humidity sensor (4), with the chemical sensor (2) and the humidity sensor (4) being arranged on the same circuit board (5),
- wherein the chemical sensor (2) and the humidity sensor (4) are each given by a sensor chip that comprises a sensing layer (6.2; 6.4) and a substrate layer (7.2; 7.4), with each substrate layer (7.2; 7.4) being provided with at least one via (8.2; 8.4) for electrically connecting the respective sensing layer (6.2; 6.4) with the circuit board (5), and
- wherein the compensation means (3) further comprise a processing unit (9) for processing the measurement signal of the chemical sensor (2) and the measurement signal of the humidity sensor (4) for error compensation, the error in the measurement signal of the chemical sensor (2) to be compensated being due to humidity.

2. Sensor arrangement according to claim 1, wherein the chemical sensor (2) and the humidity sensor (4) are arranged in close proximity or next to each other.

3. Sensor arrangement according to claim 2, wherein the lateral distance between the chemical sensor (2) and the humidity sensor (4) is smaller than or equal to 2 Millimetres.

4. Portable electronic device with a housing (10), the housing (10) being provided with an opening (11), and with a sensor arrangement (1) according to one of the preceding claims, wherein the sensor arrangement (1) is arranged inside the housing (11) beneath the opening (12) such that a fluid passing through the opening (12) comes into contact with both the chemical sensor (2) and the humidity sensor (4).
